# EUROPEAN PATENT APPLICATION

(11) **EP 4 215 121 A1**
(43) Date of publication of application: **26.07.2023**
(21) Application number: 21869696.1
(22) Date of filing: 15.09.2021
(51) Int. Cl.: A61B 10/00, C12M 1/30, G01N 1/10, A61B 10/02, G01N 1/02

(54) **SAMPLE COLLECTION STICK**

(30) Priority: 16.09.2020 KR 20200119514; 05.02.2021 KR 20210017061; 05.02.2021 KR 20210017056; 14.09.2021 KR 20210122435
(71) Applicant: Bionlifescience, Inc., Namyangju-si, Gyeonggi-do 12106 (KR)
(72) Inventor: GOH, Chang Wook, Namyangju-si Gyeonggi-do 12095 (KR); JEONG, Joong Hwan, Bucheon-si Gyeonggi-do 14500 (KR); KIM, Bong Yoon, Goyang-si Gyeonggi-do 10469 (KR)
(74) Representative: dompatent von Kreisler Selting Werner - Partnerschaft von Patent- und Rechtsanwälten mbB
(86) International application number: PCT/KR2021/012565
(87) International publication number: WO 2022/060074

(57) **Abstract**

According to the present invention, a sample collection part of a sample collection stick includes: a hub coupled to the front side of a support part; and multiple collection blades arranged on the outer surface of the hub in a shape forming an axial symmetry around the lengthwise central axis of the hub, and each of the plate-shaped collection blades is formed to, at the midpoint of the longitudinal length thereof, have a transverse width equal to or smaller than the longitudinal length and have a thickness smaller than the transverse width. Therefore, disclosed is a technique capable of increasing the amount of collected samples and increasing the amount of collected samples dissolved or dispersed into a fixing solution from a sample collection stick.

## Description

### [Technical Field]

The present invention relates to a specimen collection stick, and more particularly to a specimen collection stick capable of scraping a specimen, such as saliva in the oral cavity or the nasal cavity of a subject, thereby collecting the specimen, such as saliva in the oral cavity or the nasal cavity of the subject.

### [Background Art]

A microbiological examination is an examination for determining whether a specimen has been contaminated by disease-causing germs. That is, when it is thought that a cause of illness is a microorganism, the microbiological examination is performed in order to establish a method of diagnosing, treating, and preventing the same. The microbiological examination is performed to determine whether a subject has been contaminated by disease-causing germs, such as a colon bacillus, a typhoid bacillus, staphylococcus, and pseudomonas.

In general, a specimen is collected from the body of the subject in order to perform the microbiological examination. The collected specimen is mixed with a reagent or solution for examination, and then examination is performed to determine whether there are disease-causing germs.

In order to collect a specimen, such as saliva or a bodily fluid, from bodily tissue of the subject, a tool called a collection stick, a brush, or a swab is used. That is, the swab for specimen collection is introduced into the body of the subject, a specimen is adhered to the swab, and the swab is withdrawn, whereby the specimen is collected.

In the conventional swab for specimen collection, a circular collection unit is formed at an end of the swab, which is formed in the shape of a bar. The collection unit of the swab is provided with a fiber layer, which is formed by attaching small-sized microfibers to the collection unit. A specimen permeates the fiber layer formed on the collection unit of the conventional swab, and the collection unit of the swab is withdrawn from the subject, whereby the specimen is collected.

In the conventional swab for specimen collection used to collect the specimen, however, it is necessary for the swab to remain inserted in the oral cavity or the nasal cavity of the subject for a predetermined time or more such that saliva or the like permeates the fiber layer, whereby the subject may experience great discomfort. Above all, a part of the fiber layer included in the swab for specimen collection may be separated from the collection unit of the swab and may remain in the body of the subject. When foreign matter, such as the fiber layer, remains in the body of the subject, a medically serious problem may occur. In addition, the amount of the specimen that is collected using the conventional swab is not sufficient, whereby examination accuracy may be lowered.

Therefore, there is a need for technology capable of solving such problems.

### [Disclosure]

### [Technical Problem]

The present invention has been made in view of the above problems, and it is an object of the present invention to provide a specimen collection stick capable of increasing the amount of a specimen that is collected during specimen collection, shortening time necessary for specimen collection, and preventing a problem in which fibers constituting a fiber layer of the conventional swab are separated from the swab.

### [Technical Solution]

A specimen collection stick according to an embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, wherein the specimen collection unit includes a hub coupled to the front of the support unit and a plurality of collection blades supported by the hub, the plurality of collection blades being disposed on an outside of the hub in rotational symmetry or axial symmetry with respect to a central axis of the hub in a longitudinal direction thereof, each of the plurality of collection blades is formed in a plate shape, each of the plurality of collection blades being formed such that the longitudinal length of the collection blade from one end abutting an outer surface of the hub to the outer end thereof is equal to or greater than the lateral width of the collection blade at a center point of the longitudinal length and such that the thickness of the collection blade is less than the lateral width of the collection blade, and each of the collection blades or the hub is made of a flexible or elastic material.

The plurality of collection blades arranged at an outer circumferential surface of the hub on the same circumference may constitute a blade group, and a plurality of neighboring blade groups may be disposed on the outer circumferential surface of the hub such that each of the blade groups is spaced apart from a corresponding one of the blade groups neighboring thereto by a predetermined distance in a longitudinal direction of the hub.

The plurality of neighboring blade groups may be disposed on the outer circumferential surface of the hub such that the collection blades belonging to one of the blade groups and the collection blades belonging to another of the blade groups neighboring thereto are aligned parallel to the longitudinal direction of the hub.

Alternatively, the plurality of neighboring blade groups may be disposed on the outer circumferential surface of the hub such that the collection blades belonging to one of the blade groups are rotated relative to the collection blades belonging to another of the blade groups neighboring thereto by a predetermined angle about the central axis of the hub in the longitudinal direction thereof.

Alternatively, the plurality of collection blades may be disposed on the outer circumferential surface of the hub such that a central axis of each of the collection blades in a horizontal direction thereof parallel to a horizontal plane of each of the collection blades or the width of each of the collection blades is inclined relative to the central axis of the hub in the longitudinal direction thereof by a predetermined angle.

Each of the collection blades may be formed such that the lateral width or the thickness is gradually reduced to the other end thereof in at least a part of the longitudinal length section.

The plurality of collection blades may be spirally arranged on an outer circumferential surface of the hub along the central axis of the hub in the longitudinal direction thereof.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

A specimen collection stick according to another embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, wherein the specimen collection unit includes a hub coupled to the front of the support unit and a plurality of collection blades supported by the hub, the plurality of collection blades being disposed on an outside of the hub in rotational symmetry or axial symmetry with respect to a central axis of the hub in a longitudinal direction thereof, each of the plurality of collection blades is formed in a plate shape, each of the plurality of collection blades being formed such that the longitudinal length of the collection blade from one end abutting an outer surface of the hub to the outer end thereof is equal to or less than the lateral width of the collection blade at a center point of the longitudinal length and such that the thickness of the collection blade is less than the longitudinal length of the collection blade, and each of the collection blades or the hub is made of a flexible or elastic material.

The plurality of collection blades arranged at the outside of the hub on the same circumference may constitute a blade group, and a plurality of neighboring blade groups may be disposed on an outer circumferential surface of the hub such that each of the blade groups is spaced apart from a corresponding one of the blade groups neighboring thereto by a predetermined distance in a longitudinal direction of the hub.

The plurality of neighboring blade groups may be disposed on the outer circumferential surface of the hub such that the collection blades belonging to one of the blade groups and the collection blades belonging to another of the blade groups neighboring thereto are aligned parallel to the longitudinal direction of the hub.

Alternatively, the plurality of neighboring blade groups may be disposed on the outer circumferential surface of the hub such that the collection blades belonging to one of the blade groups are rotated relative to the collection blades belonging to another of the blade groups neighboring thereto by a predetermined angle about the central axis of the hub in the longitudinal direction thereof.

The plurality of collection blades may be disposed on the outer circumferential surface of the hub such that a central axis of each of the collection blades in a horizontal direction thereof parallel to a horizontal plane of each of the collection blades or the width of each of the collection blades is inclined relative to the central axis of the hub in the longitudinal direction thereof by a predetermined angle.

Each of the collection blades may be formed such that the lateral width or the thickness is gradually reduced to the other end thereof in at least a part of the longitudinal length section.

The plurality of collection blades may be spirally arranged on an outer circumferential surface of the hub along the central axis of the hub in the longitudinal direction thereof.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

### [Advantageous Effects]

A specimen collection stick according to the present invention has effects in that a flocking process, which is required to manufacture the conventional swab for specimen collection, is not necessary, whereby it is possible to reduce the manufacturing cost of the specimen collection stick, and in that there is no fiber layer in the specimen collection stick, whereby it is possible to prevent a problem in which the fiber layer is separated from the specimen collection stick and remains in the body of a subject, unlike the conventional swab for specimen collection, and therefore it is possible to improve examination safety.

Also, in the specimen collection stick according to the present invention, it is possible to increase the amount of the specimen that is collected from the body of the subject, and it is possible to reduce time necessary for specimen collection, since absorption time for which the specimen permeates the fiber layer is not necessary, unlike the conventional swab. Consequently, the specimen collection stick according to the present invention has effects in that it is possible to shorten time necessary for specimen collection, to reduce inconvenience of the subject during specimen collection, and to improve examination accuracy with an increase in amount of the specimen that is collected.

### [Description of Drawings]

FIG. 1 is a side view schematically showing a specimen collection stick according to an embodiment of the present invention.
FIG. 2 is a side view schematically showing a specimen collection unit of the specimen collection stick according to the embodiment of the present invention.
FIG. 3 is a front view schematically showing the specimen collection unit of the specimen collection stick according to the embodiment of the present invention.
FIG. 4 is a perspective view schematically showing the specimen collection unit of the specimen collection stick according to the embodiment of the present invention.
FIG. 5 is a view schematically showing a hub and a collection blade of the specimen collection unit of the specimen collection stick according to the embodiment of the present invention.
FIG. 6 is a side sectional conceptual view schematically showing the hub and the collection blade of the specimen collection unit of the specimen collection stick according to the embodiment of the present invention.
FIG. 7 is a side sectional conceptual view schematically showing the hub and the collection blade of the specimen collection unit of the specimen collection stick according to the embodiment of the present invention.
FIG. 8 is a side view schematically showing another example in which collection blades are disposed at the hub in the specimen collection stick according to the embodiment of the present invention.
FIG. 9 is a front view schematically showing the other example in which the collection blades are disposed at the hub in the specimen collection stick according to the embodiment of the present invention.
FIG. 10 is a perspective view schematically showing the other example in which the collection blades are disposed at the hub in the specimen collection stick according to the embodiment of the present invention.
FIGs. 11 to 13 are conceptual views schematically showing a part of the specimen collection unit of the specimen collection stick according to the embodiment of the present invention.
FIGs. 14 and 15 are conceptual views schematically showing imaginary planar development of the specimen collection unit of the specimen collection stick according to the embodiment of the present invention.
FIG. 16 is a front view schematically showing a further example in which collection blades are disposed at the hub in the specimen collection stick according to the embodiment of the present invention.
FIG. 17 is a perspective view schematically showing the further example in which the collection blades are disposed at the hub in the specimen collection stick according to the embodiment of the present invention.

### [Best Mode]

The present invention may be variously changed and may have several embodiments, and therefore specific embodiments will be described in detail while being illustrated in the drawings. However, the present invention is not limited to the specific embodiments, and it should be understood that the present invention includes all alterations, equivalents, and substitutions falling within the idea and technical scope of the present invention. The present embodiments are provided to more specifically describe the present invention to a person having ordinary skill in the art to which the present invention pertains. Consequently, the shape of each element in the drawings may be exaggerated for clearer description. In describing the present invention, a detailed description of related known technology will be omitted when the same may obscure the subject matter of the present invention.

Although the terms, such as "first" and "second," may be used to describe various elements, the elements must not be defined by the terms. The terms are used only for the purpose of distinguishing one element from another and describing the same so as to be understood.

The terms used in the present invention are used only to describe a specific embodiment, not to define the present invention. Singular forms include plural forms unless mentioned otherwise.

It should be understood that the terms "comprises," "has," etc. specify the presence of stated features, numbers, steps, operations, elements, components, or combinations thereof described in this specification, but do not preclude the presence or addition of one or more other features, numbers, steps, operations, elements, components, or combinations thereof.

Hereinafter, embodiments of the present invention will be described with reference to the accompanying drawings, and the present invention will be described in detail to the extent to which a person having ordinary skill in the art to which the present invention pertains can easily implement the present invention. However, the present invention may be implemented in various different forms and is not limited to the embodiments described herein. Similar parts may be denoted by the same reference numerals throughout the specification.

Hereinafter, embodiments of a specimen collection stick according to the present invention will be described with reference to the drawings.

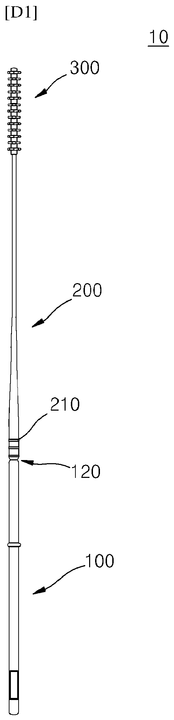

FIG. 1 is a side view schematically showing a specimen collection stick according to an embodiment of the present invention.

Referring to FIG. 1, the specimen collection stick according to the embodiment of the present invention includes a support unit 200 and a specimen collection unit 300, and may further include a handle unit 100.

First, the support unit 200 is formed so as to have a shape, such as a rod or a bar, and has a predetermined length, as shown in the figures. The specimen collection unit 300 is coupled to a front end of the support unit 200. The support unit 200 supports the specimen collection unit 300. The handle unit is connected to the rear of the support unit 200. The support unit 200 supports the specimen collection unit 300 such that the specimen collection unit can be introduced into the body of a subject.

It is sufficient for the support unit 200 to support the specimen collection unit 300 such that the specimen collection unit can be introduced into the body of the subject in order to perform specimen collection, and the shape of the support unit is not limited to a specific shape.

Furthermore, it is preferable for the support unit 200 to be made of a polymer material that exhibits flexibility and elasticity such that the support unit can return to the original shape thereof while being flexibly deformed by force applied thereto.

Meanwhile, when the specimen collection unit 300 and the support unit 200 of the specimen collection stick 10 are withdrawn from a specimen container (not shown) using a tool, such as a pincette, it is necessary to wash the pincette in advance. In addition, after the support unit 200 of the specimen collection stick 10 is withdrawn from the specimen container using the pincette and the specimen collection unit 300 and the support unit 200 of the specimen collection stick 10 are located at a position intended by an examiner, it is necessary to wash the used pincette again.

Since it is necessary to wash the pincette whenever the pincette is used in the process of withdrawing the specimen collection stick from the specimen container, as described above, specimen examination efficiency is lowered. In order to withdraw the specimen collection unit 300 and the support unit 200 of the specimen collection stick 10 from the specimen container without using the pincette, therefore, the following construction may be provided.

That is, a coupling projection or coupling protrusion 210 may be formed at an outer surface of the support unit 200 so as to protrude from the outer surface thereof such that the coupling projection or coupling protrusion is fixed to a cover or lid of the specimen container by coupling.

The cover or lid (not shown) of the specimen container (not shown) may be provided at an inside thereof with a fixing groove or fitting projection (not shown) configured to allow the support unit 200 of the specimen collection stick to be fixed to the cover or lid (not shown) of the specimen container therethrough. The coupling projection or coupling protrusion 210 of the support unit may be coupled to the fixing groove or fitting projection provided at the inside of the cover or lid of the specimen container by engagement or fitting.

Since the support unit 200 can be fixed to the cover or lid of the specimen container, as described above, it is possible to easily withdraw the support unit 200 from the specimen container together with the cover or lid of the specimen container when the cover or lid is removed from the specimen container without using a tool, such as a pincette, which is preferable.

FIG. 1 shows a ring-shaped coupling projection or coupling protrusion 210 formed on the outer surface of the support unit 200 in a circumferential direction thereof as an illustrative example of the coupling projection or coupling protrusion 210. As shown in FIG. 1, it is also preferable for one or more ring-shaped coupling projections or coupling protrusions 210 to be provided on the outer surface of the support unit 200 at the rear of the support unit 200.

As long as the support unit 200 can be coupled to the cover of the specimen container, as described above, it is possible to improve convenience in use, which is preferable.

As previously described, the handle unit 100 is located at the rear of the support unit 200. That is, a rear end of the support unit 200 is connected to the handle unit 100.

The handle unit 100 is connected to the rear end of the support unit 200, and the handle unit has a predetermined length such that the handle unit can be gripped by the hand or fingers of the examiner.

As shown in the figures, the handle unit 100 is formed in the shape of a rod or a bar having a predetermined length. The handle unit 100 is gripped by the hand or fingers of the examiner. The examiner may perform control such that the specimen collection unit 300 is introduced or inserted into the body of the subject using the handle unit 100.

Consequently, the specimen collection unit 300 may be introduced or inserted into the body of the subject through the oral cavity or the nasal cavity of the subject under control of the examiner using the handle unit 100.

As shown in the figures, it is preferable for a segment groove 120, such as a segment joint, capable of distinguishing between the handle unit 100 and the support unit 200 to be provided between the handle unit 100 and the rear end of the support unit 200. That is, it is preferable for the segment groove 120 to be provided between the handle unit 100 and the support unit 200 such that the handle unit 100 and the support unit 200 can be separated from each other.

After the specimen collection unit 300 is withdrawn from the body of the subject according to the intention of the examiner, who is a user, the specimen collection unit 300 and the support unit 200 are received in the specimen container.

Since it is not necessary to receive the handle unit 100 in the specimen container at this time, it is preferable to separate the handle unit 100 from the support unit 200. As shown in the figures, therefore, it is preferable for the segment joint or segment groove 120 to be provided between the support unit 200 and the handle unit 100 such that the support unit 200 and the handle unit 100 can be separated from each other as needed.

After the support unit 200 and the handle unit 100 are separated from each other, the support unit 200 may be coupled to the cover or lid of the specimen container, as previously described. It is preferable for the coupling projection or coupling protrusion 210 to be formed at an outer circumferential surface of the support unit so as to protrude therefrom or for a lid fastening groove to be formed in the outer circumferential surface of the support unit such that the support unit can be fixed to the cover or lid of the specimen container, in which the specimen collection unit is received, by coupling.

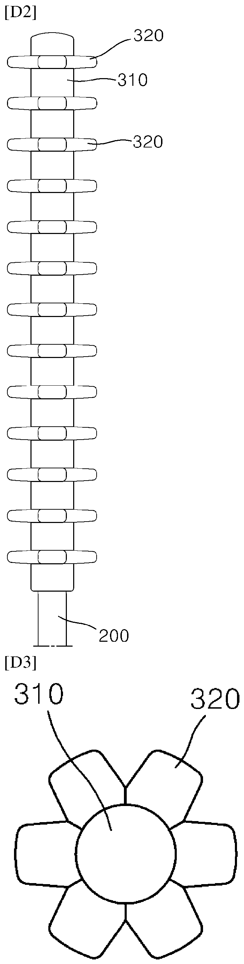

The specimen collection unit 300, which is located at the front end of the support unit 200, is a part that is inserted or introduced into the body of the subject so as to come into contact with a specimen. That is, the specimen collection unit is a part that collects the specimen. The specimen collection unit 300 will be described with further reference to FIGs. 2 to 4 for convenience of description and understanding.

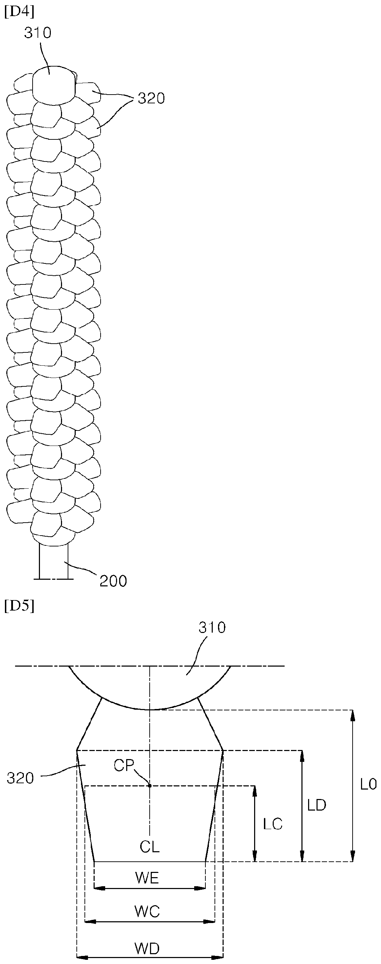

FIG. 2 is a side view schematically showing the specimen collection unit of the specimen collection stick according to the embodiment of the present invention, FIG. 3 is a front view schematically showing the specimen collection unit of the specimen collection stick according to the embodiment of the present invention, and FIG. 4 is a perspective view schematically showing the specimen collection unit of the specimen collection stick according to the embodiment of the present invention.

Referring further to FIGs. 2 to 4, the specimen collection unit 300 is located at the front end of the support unit 200 in order to gather or collect a specimen in the body of the subject that comes into contact with the specimen collection unit. The specimen collection unit 300 includes a hub 310 and a plurality of collection blades 320.

The hub 310 is coupled to the front of the support unit 200. It is preferable for the hub 310 and the support unit 200 to be coupled to each other such that a central axis of the hub in a longitudinal direction thereof and a central axis of the support unit 200 in a longitudinal direction thereof are aligned with each other. The hub 310 is coupled to or formed at the support unit 200 such that a front portion of the support unit 200 is inserted into the hub 310. In addition, the hub 310 supports the plurality of collection blades 320 disposed at the outside thereof.

The plurality of collection blades 320 is supported by the hub 310, and is disposed on an outer circumferential surface of the hub 310 in rotational symmetry or axial symmetry with respect to the central axis of the hub in the longitudinal direction thereof.

That is, the specimen collection unit 300 includes at least one collection blade 320 configured to allow a specimen brought into contact therewith to be adhered thereto or to be held thereby and to be withdrawn from the body of the subject in that state.

In addition, as shown in the figures, a part of each of the plurality of collection blades 320, i.e. one end of each of the plurality of collection blades, is formed at the hub 310 so as to be coupled thereto. The plurality of collection blades 320 thus formed is supported by the hub 310. That is, one end of each of the collection blades 320 is formed at the outer surface of the hub 310 so as to be connected thereto such that the collection blades are supported by the hub 310.

It is preferable for the hub 310 to be made of a material that exhibits flexibility or elasticity. That is, the hub may be made of a polymer material that exhibits flexibility and elasticity or an elastomer material.

The hub 310 configured to support the collection blades 320 is coupled to a part of the front of the support unit 200. The hub 310 is supported by the support unit 200. It is preferable for the central axis of the hub 310 in the longitudinal direction thereof to be aligned with the central axis of the support unit 200 in the longitudinal direction thereof.

As shown in the figures, the hub 310 may be formed in the shape of a bar having a circular section; however, the present invention is not limited thereto.

Each of the collection blades 320 is formed so as to protrude from the central axis of the hub 310 or the support unit 200 in an outward direction, i.e. a centrifugal direction, by a predetermined size such that the collection blade can come into contact with a specimen.

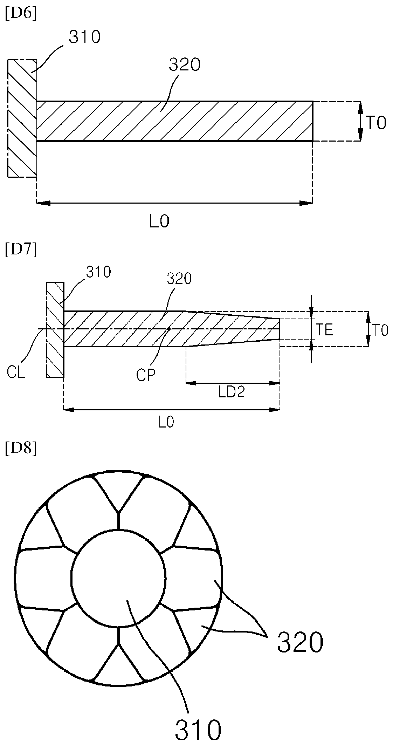

The collection blade will be descried with further reference to FIGs. 5 and 6.

FIG. 5 is a view schematically showing the hub and one collection blade of the specimen collection unit of the specimen collection stick according to the embodiment of the present invention, and FIG. 6 is a side sectional conceptual view schematically showing the hub and the collection blade of the specimen collection unit of the specimen collection stick according to the embodiment of the present invention. In FIGs. 5 and 6, only one collection blade coupled to a part of the hub is shown for convenience of description and understanding.

Each of the collection blades 320 is formed in the shape of a plate. As schematically shown in FIG. 5, each of the collection blades 320, which is formed in the shape of a plate, is formed such that the longitudinal length L0 of the collection blade from one end abutting the outer surface of the hub 310 to the outer end thereof is equal to or greater than the lateral width WC of the collection blade at the center point CP of the longitudinal length L0 and such that the thickness T0 of the collection blade is less than the lateral width WC of the collection blade.

That is, the lateral width WC of the collection blade 320 is equal to or less than the longitudinal length L0 of the collection blade 320. In addition, the thickness T0 of the collection blade is less than the lateral width WC of the collection blade.

For example, it is preferable for the longitudinal length L0 of the collection blade 320 to be one to three times the lateral width WC of the collection blade.

In FIG. 5, reference symbol LC indicates a longitudinal length section from the center point CP of the longitudinal length L0 to the other end of the collection blade, wherein LC is half the longitudinal length L0. For reference, reference symbol CL indicates a central axis of the collection blade 320 in a longitudinal direction thereof.

It is preferable for the collection blade to be formed such that at least a part LD of the longitudinal length section L0 has a lateral width (WD -> WE) gradually reduced to the other side of the collection blade.

Here, reference symbol WD indicates the lateral width WC at the point of the at least a part LD of the longitudinal length section L0 of the collection blade 320 from which the lateral width starts to be gradually reduced to the other side of the collection blade, and reference symbol WE indicates the lateral width WE at the other end of the collection blade 320.

As described above, it is preferable for the lateral width of the collection blade 320 to be reduced in order of WD > WC > WE.

It is preferable for the thickness of the collection blade 320 to be uniform from one end to the other end of the collection blade 320, as shown in FIG. 6, and it is also preferable for the thickness of a part of the collection blade to be gradually reduced, as shown in FIG. 7.

FIG. 7 is a side sectional conceptual view schematically showing the hub and the collection blade of the specimen collection unit of the specimen collection stick according to the embodiment of the present invention. In FIG. 7, only one collection blade 320 coupled to a part of the hub 310 is shown for convenience of description and understanding.

As shown in FIG. 7, the collection blade 320 may be formed such that the thickness (T0 -> TE) is gradually reduced to the other end thereof in at least a part LD2 of the longitudinal length section L0.

Here, reference symbol T0 indicates the thickness T0 at the point of the at least a part LD2 of the longitudinal length section L0 of the collection blade 320 from which the thickness starts to be gradually reduced to the other side of the collection blade, and reference symbol TE indicates the thickness TE at the other end of the collection blade 320.

That is, it is preferable for the at least a part LD2 of the longitudinal length section L0 to be formed such that the thickness is reduced from T0 to TE. In other words, it is preferable for the thickness to be tapered.

As described above, it is preferable for the collection disk 320 to be formed such that the lateral width WD or the thickness (T0 -> TE) of the at least a part LD2 of the longitudinal length section L0 is gradually reduced to the other side of the collection blade.

It is preferable for the thickness of the collection blade 320 to range from 0.1 mm to 1 mm, more preferably from 0.2 mm to 0.8 mm.

As described above, the shape of the collection blade shown in FIG. 5 is preferable, and a collection blade having the following shape may also be provided.

That is, a collection blade having a plate shape may also be formed such that the longitudinal length of the collection blade from one end abutting the outer surface of the hub to the outer end thereof is equal to or less than the lateral width of the collection blade at the center point of the longitudinal length and such that the thickness of the collection blade is less than the longitudinal length of the collection blade, which is also preferable.

That is, the longitudinal length L0 of the collection blade 320 may be equal to or less than the lateral width WC of the collection blade, and the thickness T0 of the collection blade may be less than the longitudinal length of the collection blade.

For example, it is preferable for the lateral width of the collection blade 320 to be one to two times the longitudinal length of the collection blade.

The collection blade 320 may be formed in the shape of a plate having at least one curved or flat side surface. That is, at least a part of the surface of the collection blade 320 may be curved or flat. Also, it is preferable for outer corners of the collection blade having the plate shape to be rounded or curved.

When the outer corners of the collection blade 320 having the plate shape are rounded or curved, it is possible to inhibit a possibility of a wound occurring in the body of the subject during the collection process, which is preferable.

As previously described, it is preferable for the collection blade 320 or the hub 310 to be made of a flexible or elastic material. That is, the collection blade 320 or the hub 310 may be made of a polymer material that exhibits flexibility and elasticity or an elastomer material.

As shown in the figures, the plurality of collection blades 320 is disposed or arranged at the outer circumferential surface of the hub 310 on the same circumference. FIGs. 2 and 3 show an example in which six collection blades 320 are disposed on the same circumference; however, the number of the collection blades 320 is not particularly restricted.

The plurality of collection blades 320 disposed or arranged at the outer circumferential surface of the hub 310 on the same circumference may constitute a blade crew or a blade group. For reference, the center of the circumference is located on the central axis of the hub in the longitudinal direction thereof.

As shown in FIG. 2 or 4, a plurality of blade groups is disposed so as to be spaced apart from each other by a predetermined distance in the central axis direction of the hub 310.

The plurality of blade groups may be sequentially arranged along the central axis of the hub 310 in the longitudinal direction thereof.

It is preferable for the plurality of neighboring blade groups to be disposed on the outer circumferential surface of the hub 310 such that each of the blade groups is spaced apart from a corresponding one of the blade groups neighboring thereto by a predetermined distance in the longitudinal direction of the hub. Of course, it is also preferable for the distance between the blade groups to be uniform.

As shown in FIG. 2, the neighboring blade groups are spaced apart from each other. A specimen entering a space between the blade groups spaced apart from each other comes into contact with or is adhered to the surface of each of the collection blades 320 or the surface of the hub 310. As a result, it is possible to achieve an effect in that the specimen is held by the surfaces of the collection blades 320 and the surface of the hub 310 in the vicinity of the specimen.

In addition, as shown in FIGs. 8 to 10, an applied example may be provided.

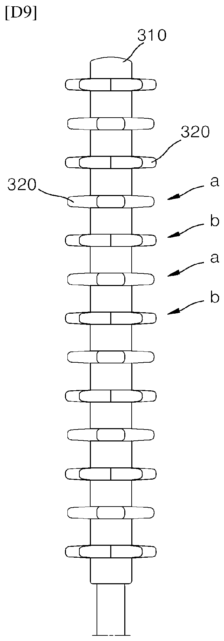

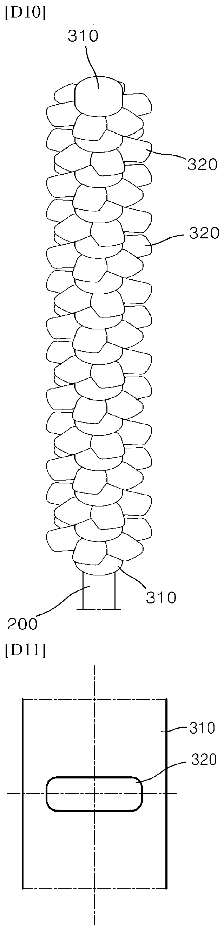

FIG. 8 is a side view schematically showing another example in which the collection blades are disposed at the hub in the specimen collection stick according to the embodiment of the present invention, FIG. 9 is a front view schematically showing the other example in which the collection blades are disposed at the hub in the specimen collection stick according to the embodiment of the present invention, and FIG. 10 is a perspective view schematically showing the other example in which the collection blades are disposed at the hub in the specimen collection stick according to the embodiment of the present invention.

As shown in FIGs. 8 to 10, the disposition of the collection blades 320 in one blade group is different from the disposition of the collection blades 320 in another blade group neighboring thereto.

That is, as shown in FIGs. 2 to 4, each of the collection blades 320 in one blade group is aligned with a corresponding one of the collection blades 320 in another blade group neighboring thereto so as to form a line. In FIGs. 8 to 10, however, the collection blades 320 are not aligned with each other but are rotated by a predetermined angle about the central axis of the hub 310 in the longitudinal direction thereof.

As described above, the positions at which the collection blades 320 are formed in the blade groups may be different from each other, which is preferable.

As shown in FIG. 8, the collection blades 320 in the blade group indicated by reference symbol a and the collection blades 320 in the blade group indicated by reference symbol b are rotated by a predetermined angle about the central axis of the hub 310 in the longitudinal direction thereof so as to be aligned with each other in axial symmetry or rotational symmetry.

In addition, as shown in FIGs. 8 to 10, the blade group indicated by reference symbol a and the blade group indicated by reference symbol b are alternately disposed. Since the blade groups, in which the pluralities of collection blades 320 are coupled to the hub 310 in different directions, are alternately disposed in an axial direction of the hub 320, as described above, it is possible to improve specimen collection efficiency, which is preferable.

Next, the structure in which the collection blade is coupled to the hub will be described with reference to FIGs. 11 to 13.

FIGs. 11 to 13 are conceptual views schematically showing a part of the specimen collection unit of the specimen collection stick according to the embodiment of the present invention, i.e. the structure in which the collection blade 320 is coupled to the hub 310.

For convenience of understanding and description, only one collection blade 320 is shown as being coupled to the hub 310 in FIGs. 11 to 13.

As shown in FIG. 11, the collection blade 320 may be coupled to the hub 310 such that a central axis of the collection blade in a horizontal direction thereof is perpendicular to the central axis of the hub in the longitudinal direction thereof. Here, the central axis of the collection blade in the horizontal direction thereof is identical or parallel to the direction of the lateral width WC previously described with reference to FIG. 5.

In addition, the central axes of the plurality of collection blades in the horizontal direction thereof may be inclined relative to the central axis of the hub in the longitudinal direction thereof by a predetermined angle, which is preferable.

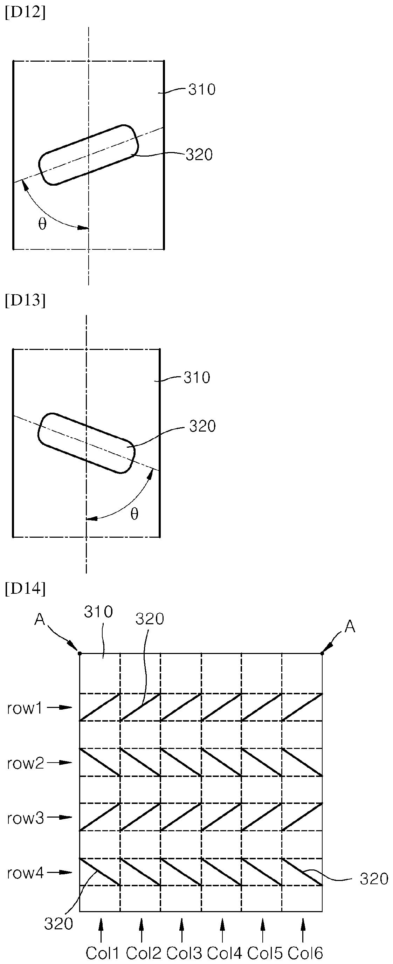

As shown in FIG. 12, the collection blade 320 may be formed at the surface of the hub 310 such that the central axis of the collection blade 320 in the horizontal direction thereof is inclined relative to the central axis of the hub 310 in the longitudinal direction thereof upwards in a rightward direction by a predetermined angle θ, which is also preferable. As shown in FIG. 13, the collection blade 320 may be formed at the surface of the hub 310 such that the central axis of the collection blade 320 in the horizontal direction thereof is inclined relative to the central axis of the hub 310 in the longitudinal direction thereof downwards in the rightward direction by a predetermined angle θ, which is also preferable.

As described above, the plurality of collection blades 320 may be coupled to the hub 310 such that a horizontal axis or horizontal plane of each of collection blades is inclined relative to the central axis of the hub in the longitudinal direction thereof by a predetermined angle, which is also preferable.

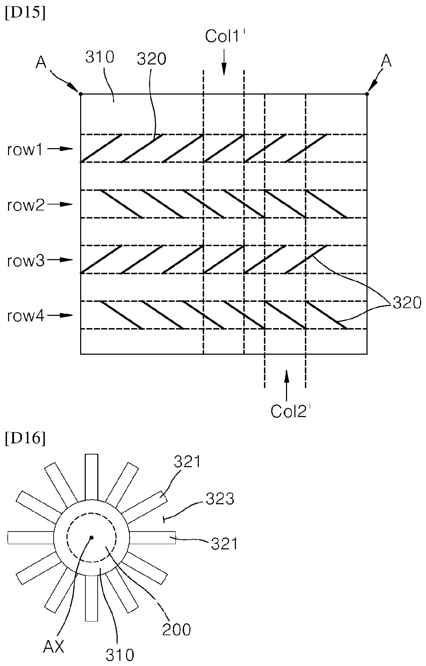

FIGs. 14 and 15 are conceptual views schematically showing imaginary planar development of the specimen collection unit of the specimen collection stick according to the embodiment of the present invention.

For convenience of understanding and description, an illustrative example in which six collection blades 320 are disposed on the same row to constitute one blade group and four blade group are disposed is schematically shown, wherein the planar shape of the specimen collection unit when the outer surface of the hub 310, the longitudinal section of which is circular, is developed in a planar shape is schematically shown.

Here, reference symbol A of FIGs. 14 and 15 indicates a predetermined point on the surface of the hub 310, and when the hub is bent such that a left point A and a right point A come into contact with each other, the shape of the hub is formed as previously described.

First, as shown in FIG. 14, six collection blades 320 belonging to a first row row1 constitute one blade group. In addition, six collection blades 320 belonging to each of a second row row2, a third row row3, and a fourth row row4 constitute one blade group.

Each of the six collection blades 320 belonging to the first row row1 and the six collection blades 320 belonging to the third row row3 is coupled to the hub 310 such that each collection blade 320 is inclined relative to the central axis of the hub upwards in the rightward direction by a predetermined angle, as previously described with reference to FIG. 12.

In addition, each of the six collection blades 320 belonging to the second row row2 and the six collection blades 320 belonging to the fourth row row4 is coupled to the hub 310 such that each collection blade 320 is inclined relative to the central axis of the hub downwards in the rightward direction by a predetermined angle, as previously described with reference to FIG. 13.

As described above, the collection blades 320 may be disposed on the surface of the hub 310 such that the direction in which the collection blades 320 belonging to one blade group are inclined and the direction in which the collection blades 320 belonging to another blade group neighboring thereto are inclined are different from each other, as shown in FIG. 14 or 15, which is preferable.

As described above, it is also preferable for the inclination angle of the collection blades 320 belonging to one blade group and the inclination angle of the collection blades 320 belonging to another blade group neighboring thereto to be different from each other.

In addition, as shown in FIG. 14, each of the collection blades 320 commonly belongs to one column. That is, the collection blades 320 are disposed in a state of being aligned in the longitudinal direction of the central axis of the hub 310.

As shown in FIG. 15, which shows an applied example of FIG. 14, each of the six collection blades 320 belonging to the first row row1 and the six collection blades 320 belonging to the third row row3 is coupled to the hub 310 such that each collection blade 320 is inclined relative to the central axis of the hub upwards in the rightward direction by a predetermined angle, as previously described with reference to FIG. 12.

In addition, one of the collection blades 320 belonging to the first row row1 and one of the collection blades 320 belonging to the third row row3 are aligned with each other on one column, such as a column indicated by reference symbol Col1'.

In addition, each of the six collection blades 320 belonging to the second row row2 and the six collection blades 320 belonging to the fourth row row4 is coupled to the hub 310 such that each collection blade 320 is inclined relative to the central axis of the hub downwards in the rightward direction by a predetermined angle, as previously described with reference to FIG. 13. Here, one of the collection blades 320 belonging to the second row row2 and one of the collection blades 320 belonging to the fourth row row4 are aligned with each other in one column, such as a column indicated by reference symbol Col2'.

In addition, the collection blades 320 belonging to the first row row1 and the collection blades 320 belonging to the second row row2 are not aligned with each other. This arrangement of the collection blades 320 is possible and preferable.

Since the hub 310 and the plurality of collection blades 320 are provided, as described above, the surface area of the specimen collection unit 300 is greater than the surface area of the conventional swab, as shown in the figures.

In addition, as previously described, each of the plurality of collection blades 320 is flexible and elastic. Consequently, each of the plurality of collection blades 320 abuts bodily tissue in the body of the subject. The examiner swings the specimen collection unit 300 in the body of the subject using the handle unit 100 such that the collection blades 320 can scrape or sweep the bodily tissue in which the specimen is present while abutting the bodily tissue. As a result, the specimen is adhered to the surface of each of the collection blades 320 or is held in a gap between neighboring ones of the collection blades 320.

That is, the specimen is adhered to the surface of each of the collection blades 320 or is held in the gap between the neighboring ones of the collection blades 320 in the state in which the specimen collection unit 300 is inserted in the body of the subject, whereby the specimen remains on the specimen collection unit 300 when the specimen collection unit 300 is withdrawn from the body of the subject. The gap between the neighboring ones of the collection blades 320 may also be referred to as a collection space or a collection gap.

It is preferable for the collection blades 320 to be manufactured by molding such that each of the collection blades has a size set depending on the part of the body of the subject into which the collection disks are inserted, such as the nasal cavity, the oral cavity, the anus, or the cervix. The specimen collection unit 300 including the collection blades 320 and the hub 310 may be made of a polymer material that exhibits flexibility and elasticity or an elastomer.

In the specimen collection stick according to the embodiment of the present invention, as described above, the amount of the specimen that is collected by the plurality of collection blades 320 included in the specimen collection unit 300 is greater than the amount of the specimen collected by the conventional swab for specimen collection.

Consequently, it is possible to increase the amount of the specimen that is collected from the subject and to increase the amount of the collected specimen that is dissolved or dispersed in a reagent or a solution from the specimen collection stick.

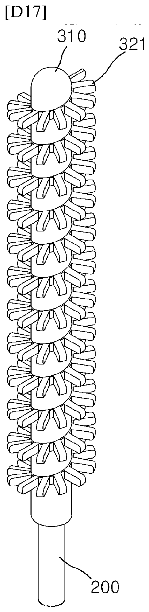

An applied embodiment may also be provided, as shown in FIGs. 16 and 17.

FIG. 16 is a front view schematically showing a further example in which collection blades are disposed at the hub in the specimen collection stick according to the embodiment of the present invention, and FIG. 17 is a perspective view schematically showing the further example in which the collection blades are disposed at the hub in the specimen collection stick according to the embodiment of the present invention.

As shown in FIGs. 16 and 17, a plurality of collection blades 321 may be spirally arranged on the outer circumferential surface of the hub 310 along the central axis AX of the hub 310 in the longitudinal direction thereof.

A specimen may be adhered and held in a gap between neighboring ones of the plurality of collection blades 321 arranged as described above. Consequently, it is preferable for the plurality of collection blades 321 to be spirally arranged on the outer circumferential surface of the hub 310.

FIGs. 16 and 17 show by way of example that the lateral width of each of the collection blades 321 is greater than the longitudinal length thereof; however, the present invention is not limited thereto. The longitudinal length of each of the collection blades may be greater than the lateral width thereof.

As is apparent from the above description, in the specimen collection stick according to the embodiment of the present invention, the amount of the specimen that is collected by the plurality of collection blades included in the specimen collection unit and in the gap between neighboring ones of the collection blades is greater than the amount of the specimen collected by the conventional swab for specimen collection.

As can be seen from the above description, the specimen collection stick according to the present invention has advantages in that a flocking process, which is required to manufacture the conventional swab for specimen collection, is not necessary, whereby it is possible to reduce the manufacturing cost of the specimen collection stick, and in that there is no fiber layer in the specimen collection stick, whereby it is possible to prevent a problem in which the fiber layer is separated from the specimen collection stick and remains in the body of the subject, unlike the conventional swab for specimen collection.

In addition, the specimen collection stick according to the present invention has advantages in that it is possible to increase the amount of the specimen that is collected from the subject, compared to the conventional swab, to increase the amount of the collected specimen that is dissolved or dispersed in a reagent or a solution from the specimen collection stick, and to improve examination accuracy with an increase in amount of the specimen that is collected.

Furthermore, the specimen collection stick according to the present invention has advantages in that it is possible to shorten time necessary for specimen collection and to reduce inconvenience of the subject during specimen collection. In addition, the specimen collection unit, which is inserted into the oral cavity or the nasal cavity of the subject, is made of a polymer material that exhibits flexibility and elasticity or an elastomer material, whereby it is possible to reduce inconvenience of the user.

Although the present invention has been described in detail based on the embodiment with reference to the accompanying drawings, as described above, the embodiments are merely provided to describe the present invention, and therefore it should be understood that the present invention is not limited to the embodiments, and the scope of rights of the present invention should be interpreted by the following claims and equivalent thereto.

- 100:: Handle unit
- 200:: Support unit
- 300:: Specimen collection unit
- 310:: Hub
- 320:: Collection blade

## Claims

1. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, wherein
the specimen collection unit comprises:
a hub coupled to a front of the support unit; and
a plurality of collection blades supported by the hub, the plurality of collection blades being disposed on an outside of the hub in rotational symmetry or axial symmetry with respect to a central axis of the hub in a longitudinal direction thereof,
each of the plurality of collection blades is formed in a plate shape, each of the plurality of collection blades being formed such that a longitudinal length of the collection blade from one end abutting an outer surface of the hub to the outer end thereof is equal to or greater than a lateral width of the collection blade at a center point of the longitudinal length and such that a thickness of the collection blade is less than the lateral width of the collection blade, and
each of the collection blades or the hub is made of a flexible or elastic material.

2. The specimen collection stick according to claim 1, wherein
the plurality of collection blades arranged at an outer circumferential surface of the hub on the same circumference constitute a blade group, and
a plurality of neighboring blade groups is disposed on the outer circumferential surface of the hub such that each of the blade groups is spaced apart from a corresponding one of the blade groups neighboring thereto by a predetermined distance in a longitudinal direction of the hub.

3. The specimen collection stick according to claim 2, wherein the plurality of neighboring blade groups is disposed on the outer circumferential surface of the hub such that the collection blades belonging to one of the blade groups and the collection blades belonging to another of the blade groups neighboring thereto are aligned parallel to the longitudinal direction of the hub.

4. The specimen collection stick according to claim 2, wherein the plurality of neighboring blade groups is disposed on the outer circumferential surface of the hub such that the collection blades belonging to one of the blade groups are rotated relative to the collection blades belonging to another of the blade groups neighboring thereto by a predetermined angle about the central axis of the hub in the longitudinal direction thereof.

5. The specimen collection stick according to claim 2, wherein the plurality of collection blades is disposed on the outer circumferential surface of the hub such that a central axis of each of the collection blades in a horizontal direction thereof parallel to a horizontal plane of each of the collection blades or a width of each of the collection blades is inclined relative to the central axis of the hub in the longitudinal direction thereof by a predetermined angle.

6. The specimen collection stick according to claim 1, wherein each of the collection blades is formed such that the lateral width or the thickness is gradually reduced to the other end thereof in at least a part of the longitudinal length section.

7. The specimen collection stick according to claim 1, wherein the plurality of collection blades is spirally arranged on an outer circumferential surface of the hub along the central axis of the hub in the longitudinal direction thereof.

8. The specimen collection stick according to claim 1, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.

9. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, wherein
the specimen collection unit comprises:
a hub coupled to a front of the support unit; and
a plurality of collection blades supported by the hub, the plurality of collection blades being disposed on an outside of the hub in rotational symmetry or axial symmetry with respect to a central axis of the hub in a longitudinal direction thereof,
each of the plurality of collection blades is formed in a plate shape, each of the plurality of collection blades being formed such that a longitudinal length of the collection blade from one end abutting an outer surface of the hub to the outer end thereof is equal to or less than a lateral width of the collection blade at a center point of the longitudinal length and such that a thickness of the collection blade is less than the longitudinal length of the collection blade, and
each of the collection blades or the hub is made of a flexible or elastic material.

10. The specimen collection stick according to claim 9, wherein
the plurality of collection blades arranged at the outside of the hub on the same circumference constitute a blade group, and
a plurality of neighboring blade groups is disposed on an outer circumferential surface of the hub such that each of the blade groups is spaced apart from a corresponding one of the blade groups neighboring thereto by a predetermined distance in a longitudinal direction of the hub.

11. The specimen collection stick according to claim 10, wherein the plurality of neighboring blade groups is disposed on the outer circumferential surface of the hub such that the collection blades belonging to one of the blade groups and the collection blades belonging to another of the blade groups neighboring thereto are aligned parallel to the longitudinal direction of the hub.

12. The specimen collection stick according to claim 10, wherein the plurality of neighboring blade groups is disposed on the outer circumferential surface of the hub such that the collection blades belonging to one of the blade groups are rotated relative to the collection blades belonging to another of the blade groups neighboring thereto by a predetermined angle about the central axis of the hub in the longitudinal direction thereof.

13. The specimen collection stick according to claim 10, wherein the plurality of collection blades is disposed on the outer circumferential surface of the hub such that a central axis of each of the collection blades in a horizontal direction thereof parallel to a horizontal plane of each of the collection blades or a width of each of the collection blades is inclined relative to the central axis of the hub in the longitudinal direction thereof by a predetermined angle.

14. The specimen collection stick according to claim 9, wherein each of the collection blades is formed such that the lateral width or the thickness is gradually reduced to the other end thereof in at least a part of the longitudinal length section.

15. The specimen collection stick according to claim 9, wherein the plurality of collection blades is spirally arranged on an outer circumferential surface of the hub along the central axis of the hub in the longitudinal direction thereof.

16. The specimen collection stick according to claim 9, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.
